# EUROPÄISCHE PATENTSCHRIFT

(11) **EP 4 384 103 B1**
(45) Veröffentlichungstag und Bekanntmachung des Hinweises auf die Patenterteilung: **12.03.2025**
(21) Anmeldenummer: 23722585.9
(22) Anmeldetag: 05.05.2023
(51) Int. Cl.: A61B 34/10, A61B 5/11, A61F 2/30, B33Y 50/00, G06F 30/17, G16H 20/40, G16H 30/40, G16H 40/63, G16H 50/20, G16H 50/50, A61B 17/00, A61F 2/38, B33Y 30/00, A61F 2/46, G06F 111/16, G06F 113/10

(54) **SYSTEM UND VERFAHREN ZUR PRÄ-OPERATIVEN PLANUNG EINER KNIEENDOPROTHESE**
SYSTEM AND METHOD FOR PREOPERATIVELY PLANNING A KNEE ENDOPROSTHESIS
SYSTÈME ET MÉTHODE DE PLANIFICATION PRÉOPÉRATOIRE D'UNE ENDOPROTHÈSE DU GENOU

(30) Priorität: 06.05.2022 DE 102022111282
(43) Veröffentlichungstag der Anmeldung: 19.06.2024
(73) Patentinhaber: Aesculap AG, 78532 Tuttlingen (DE)
(72) Erfinder: UTZ, Michael, 78532 Tuttlingen (DE); MAAS, Allan, 78467 Konstanz (DE); DUPRAZ, Ingrid, 78532 Tuttlingen (DE); GRUPP, Thomas, 78588 Denkingen (DE)
(74) Vertreter: Winter, Brandl - Partnerschaft mbB
(86) Internationale Anmeldenummer: PCT/EP2023/061987
(87) Internationale Veröffentlichungsnummer: WO 2023/214042

(56) Entgegenhaltungen:
- WO-A1-2017/196817
- WO-A1-2020/163358
- WO-A1-2021/008892
- US-A1- 2014 222 157
- US-A1- 2014 222 390
- US-A1- 2019 380 792

## Beschreibung

Die vorliegende Offenbarung betrifft ein System bzw. eine Vorrichtung zur prä-operativen Planung oder Auswahl eines Implantattyps und einer Implantatgröße undr einer Implantatform einer Knie(total)endoprothese, das heißt für einen totalen Kniegelenkersatz (auch engl. "Total Knee Arthoplasty (TKA)" bezeichnet). Ferner betrifft die Offenbarung eine entsprechendes Verfahren zur prä-operativen Planung oder Auswahl einer Knieendoprothese.

### Stand der Technik

Die prä-operative Planung von Knieendoprothesen erfolgt in bekannter Weise anhand von Bilddaten, welche die Anatomie, also die statische Gestalt, Lage und Struktur des Kniegelenks, welches den Oberschenkelknochen (Femur) mit dem Schienbeinknochen (Tibia) und der Kniescheibe (Patella) zusammen zu einem Gelenk verbindet, abbilden. Beispielsweise existieren 2D- oder 3D-Planungs- bzw. Auswahlsysteme, mittels welchen auf der Grundlage von Röntgenbildern, Computertomographie-Daten oder Magnetresonanztomographie-Daten Knieendoprothesen im Hinblick auf Implantattyp, Implantatgröße und/oder Implantatform geplant bzw. ausgewählt werden können.

In Studien hat sich herausgestellt, dass ein relevanter Anteil der Patienten, deren Knieendoprothese mit einem existierenden 2D- oder 3D-Planungs- bzw. Auswahlsystem geplant wurde und die im Nachhinein eine entsprechende Knieendoprothese erhalten haben, mit ihrer Knieendoprothese unzufrieden sind. Insbesondere führen Knieendoprothesen bei diesen Patienten zu einem unnatürlichen Gelenksgefühl und zu Bewegungseinschränkungen oder verursachen sogar Schmerzen. Darüber hinaus berichten diese Patienten auch von frühzeitigen Abnutzungen und Lockerungen des Implantats.

US 2014/222390 A1 offenbart Methoden und Vorrichtungen für ein prä-operatives Designen und Auswählen von Implantaten oder Implantatkomponenten für das Kniegelenk.

WO 2017/196817 A1 offenbart eine prä-operative Methode für das Herstellen von angepassten Implantatkomponenten. Dabei wird nicht das gesamte Implantat angepasst. Die femorale Grundplatte und die tibiale Grundplatte werden unverändert als Standardbauteile verwendet.

WO 2021/008892 A1offenbart eine Methode für das Designen von zweiteiligen Gelenkprothesen.

US 2014/222157 A1 offenbart Methoden für ein Erhalten von kinematischen Informationen bezüglich eines Gelenks bei Gewichtsbelastung und Methoden für ein Designen von Implantaten basierend auf den erhaltenen kinematischen Informationen.

WO 2020/163358 A1 offenbart ein Verfahren und System für einen arthroplastischen Eingriff z.B. am Knie. Dabei wird das Gelenk präoperativ in einer Vielzahl von Beugungsstellungen analysiert und basierend darauf mittels eines statischen Patientenmodells die Performance eines Kniegelenks vorhergesagt.

US 2019/0380792 A1 offenbart eine Methode zur virtuellen Führung für orthopädische, chirurgische Eingriffe. Dazu gehört ein überlagertes Anzeigen eines virtuellen Modells eines Körperteils aus einem chirurgischen Plans auf dem Körperteil, um eine Prothese anzubringen, und eine Anzeige zum Vorbereiten des Körperteils und/oder Anbringen der Prothese an das Körperteil.

### Kurzbeschreibung der Offenbarung

Aufgabe der vorliegenden Offenbarung ist es, die Nachteile des Standes der Technik zu vermeiden oder wenigstens zu mildern. Insbesondere soll eine Planung und eine Auswahl von Knieendoprothesen derart verbessert werden, dass bei möglichst vielen Patienten, welche eine Knieendoprothese erhalten, die Knieendoprothese gut funktioniert und sich ein natürliches Gelenksgefühl einstellt. Insbesondere sollen möglichst viele Patienten keine Bewegungseinschränkungen oder Schmerzen empfinden. Darüber hinaus soll es auch nicht zu frühzeitigen Abnutzungen und Lockerungen des Implantats kommen.

Diese Aufgabe wird durch ein System bzw. eine Vorrichtung zur prä-operativen Planung oder Auswahl einer Knieendoprothese nach Anspruch 1 und ein Verfahren zur prä-operativen Planung oder Auswahl einer Knieendoprothese nach Anspruch 10 gelöst. Vorteilhafte Ausführungsformen und Weiterbildungen sind in den Unteransprüchen beansprucht und/oder werden nachfolgend erläutert.

Die Offenbarung betrifft zunächst ein System bzw. eine Vorrichtung zur prä-operativen Planung oder Auswahl eines Implantattyps (einer Implantatart) und einer Implantatgröße und einer Implantatform einer Knie(total)endoprothese, das heißt eines totalen Kniegelenkersatzes ("Total Knee Arthoplasty TKA"), mit: einer Datenbereitstellungseinheit, welche eingerichtet ist, zumindest patientenspezifische bzw. patientenindividuelle kinematische Daten bereitzustellen, welche bei einer Flexion bzw. Beugung eines Knies bzw. Kniegelenks eines Patienten erhalten werden, und einer Steuereinheit, welche eingerichtet ist, die von der Datenbereitstellungseinheit bereitgestellten kinematischen Daten zu verarbeiten und den Implantattyp und die Implantatgröße und die Implantatform unter Berücksichtigung zumindest der kinematischen Daten, das heißt auf der Grundlage von zumindest den kinematischen Daten zu bestimmen, wobei die Steuereinheit eingerichtet ist zu bestimmen, dass eine patientenspezifische prä-operative Kinematik des Kniegelenks am besten mit einem sogenannten Medial Pivot Implantat nachgebildet werden kann, und zu bestimmen, dass, falls die patientenspezifische prä-operative Kinematik des Patienten einen ausgeprägten femoralen Roll-Back zeigt, die Kinematik des Kniegelenks am besten mit einem sogenannten PS-Implantat nachgebildet werden kann, welches einen derartigen femoralen Roll-Back erzwingt, unterstützt und fördert.

In anderen Worten wird gemäß der vorliegenden Offenbarung bei der prä-operativen Planung oder Auswahl der Knieendoprothese die Flexions- bzw. Beugebewegung des Kniegelenks und somit ein tatsächlicher prä-operativer kinematischer Bewegungsablauf in dem Kniegelenk des Patienten berücksichtigt, und der Implantattyp und die Implantatgröße und die Implantatform werden derart bestimmt bzw. ausgewählt, dass post-operativ durch die implantierte Knieendoprothese der prä-operative kinematische Bewegungsablauf und somit die prä-operative Kinematik möglichst gut nachgebildet wird. Der vorliegenden Offenbarung liegt somit der Kerngedanke zugrunde, dass der kinematische Phänotyp, also das kinematische Erscheinungsbild des Patienten, insbesondere der Bewegungsablauf des Patienten im Kniegelenk bei der Planung bzw. Auswahl der Knieendoprothese berücksichtigt wird.

Unter einer prä-operativen Planung oder Auswahl ist offenbarungsgemäß eine Planung oder Auswahl vor einem Operationsvorgang zu verstehen. Eine intra-operative Anpassung bzw. Planung der Knieendoprothese ist somit nicht Gegenstand der vorliegenden Offenbarung. Die prä-operative Planung oder Auswahl ist somit vor einer Operation des Patienten abgeschlossen, das heißt die offenbarungsgemäße Vorrichtung bzw. insbesondere deren Steuereinheit ist eingerichtet, den Implantattyp und die Implantatgröße und die Implantatform basierend auf zumindest den kinematischen Daten vor der Operation des Patienten zu bestimmen.

Die Berücksichtigung der kinematischen Daten kann derart erfolgen, dass sie die Implantatgröße bestimmen bzw. beeinflussen. Beispielsweise kann von der Steuereinheit in Abhängigkeit von der Kinematik im Kniegelenk das Implantat geeignet skaliert werden, damit die Größe des Implantats bestmöglich an den entsprechenden Patienten angepasst ist. Zusätzlich oder alternativ kann die Berücksichtigung der kinematischen Daten derart erfolgen, dass sie die Implantatform bestimmen bzw. beeinflussen. Die Implantatform ist beispielsweise durch die Implantatfamilie/ Produktfamilie bestimmt. Alternativ oder zusätzlich sind unter der Implantatform beispielsweise auch lokale, abschnittsweise, das heißt bereichsweise Anpassungen bzw. Änderungen, beispielsweise Verstärkungen des Implantats zu verstehen. Es kann etwa vorgesehen sein, dass unter Berücksichtigung der kinematischen Daten das Implantat lokal verstärkt wird, beispielsweise indem lokal bzw. abschnittsweise mehr Material verwendet wird oder indem Verstärkungsstrukturen wie etwa Rippen lokal bzw. abschnittsweise vorgesehen werden. Zusätzlich oder alternativ kann die Berücksichtigung der kinematischen Daten derart erfolgen, dass sie den Implantattyp bzw. die Implantatart bestimmen bzw. beeinflussen. Eine Implantatfamilie/ Produktfamilie kann verschiedene Implantattypen/ Implantatarten enthalten. Beispielsweise kann die Steuereinheit bestimmen, dass die patientenspezifische prä-operative Kinematik des Kniegelenks am besten mit einem sogenannten Medial Pivot Implantat nachgebildet werden kann. Falls die natürliche, prä-operative Kinematik des Patienten einen ausgeprägten femoralen Roll-Back zeigt, kann die Steuereinheit beispielsweise bestimmen, dass die Kinematik des Kniegelenks am besten mit einem sogenannten PS-Implantat nachgebildet werden kann, welches einen derartigen femoralen Roll-Back erzwingt, unterstützt und fördert.

Es ist von Vorteil, wenn zumindest zwei Einflussgrößen aus dem Implantattyp, der Implantatgröße und der Implantatform von der Steuereinheit bestimmt bzw. beeinflusst werden, beispielsweise der Implantattyp und die Implantatgröße. Ein besonders bevorzugtes Ausführungsbeispiel ist dadurch gekennzeichnet, dass sowohl Implantattyp als auch Implantatgröße als auch Implantatform von der Steuereinheit bestimmt bzw. beeinflusst werden. Besonders bevorzugt werden Implantatgröße, Implantatfamilie und Implantattyp von der Steuereinheit bestimmt.

Die Datenbereitstellungseinheit kann eine Eingabeeinheit und/oder eine Erfassungseinheit aufweisen. In anderen Worten kann die Datenbereitstellungseinheit eine Eingabeeinheit oder eine Erfassungseinheit oder sowohl eine Eingabeeinheit als auch eine Erfassungseinheit aufweisen. Es kann vorgesehen sein, dass die kinematischen Daten von einem Anwender über die Eingabeeinheit (manuell) eingegeben werden können. Alternativ oder zusätzlich kann vorgesehen sein, dass die kinematischen Daten über die Erfassungseinheit erfasst werden.

Die Eingabeeinheit kann eine Benutzerschnittstelle sein und die kinematischen Daten können über ein Eingabegerät der Benutzerschnittstelle, beispielsweise über eine Tastatur, eine Maus, einen Touchscreen, Spracherkennung, etc., von dem Anwender eingegeben werden. Beispielsweise kann der Anwender eine Beweglichkeit des Knies/ Kniegelenks prä-operativ untersuchen und aus dieser Untersuchung gewonnene bzw. erhaltene kinematische Daten in das Eingabegerät eingeben. Von Vorteil ist dabei, dass die Beweglichkeit bzw. Beugung des Knies/ Kniegelenks bei der manuellen prä-operativen Untersuchung über den gesamten Bewegungsumfang, das heißt von größtmöglicher Extension in größtmögliche Flexion und zurück, betrachtet wird und somit kinematische Daten über den gesamten Bewegungsumfang erhalten werden. Wenn eine Schädigung des zu operierenden Kniegelenks eine adäquate Bestimmung einer natürlichen Kinematik nicht mehr zulässt, kann auch das kontralaterale Kniegelenk untersucht werden und die für das Kniegelenk auf der kontralateralen Seite gewonnenen Daten verwendet werden. In anderen Worten kann bei einer Schädigung beispielsweise des linken Kniegelenks das rechte Kniegelenk untersucht werden oder andersherum. Die Datenbereitstellungseinheit kann die über die Eingabeeinheit erhaltenen kinematischen Daten der Steuereinheit zur weiteren Verarbeitung bereitstellen.

Die Erfassungseinheit kann eine Kamera sein oder zumindest eine Kamera aufweisen, welche eingerichtet ist, die kinematischen Daten zu erfassen. Beispielsweise kann die Erfassungseinheit bzw. die Kamera ein physiologisches Gangbild eines Patienten erfassen oder aufzeichnen. Das physiologische Gangbild eines Patienten kann nach Jacquelin Perry in einzelne (Gang-)Phasen eines Doppelschrittes eingeteilt werden, wobei sich die Flexion bzw. Beugung des Knies bzw. Kniegelenks kontinuierlich über den zeitlichen Ablauf der Gangphasen des Doppelschrittes ändert. Es können somit über die zumindest eine Kamera patientenspezifische bzw. patientenindividuelle kinematische Daten erfasst werden, welche bei einer Flexion bzw. Beugung eines Knies eines Patienten, insbesondere über den zeitlichen Ablauf der Gangphasen des Doppelschrittes, erhalten werden. Es können grundsätzlich auch mehrere Kameras vorgesehen sein, um die kinematischen Daten aus verschiedenen Richtungen, beispielsweise von vorne, von hinten, von einer Seite, etc. zu erfassen. Gemäß diesem Ausführungsbeispiel wird somit die Flexion bzw. Beugung des Knies nicht über den gesamten Bewegungsumfang, sondern nur über das Gangbild des Patienten, das heißt den zeitlichen Ablauf der Gangphasen des Doppelschrittes erfasst. Auch über diesen begrenzten Bewegungsumfang erhaltene kinematische Daten reichen grundsätzlich aus, um insbesondere bei älteren Patienten durch Bereitstellung dieser kinematischen Daten von der Datenbereitstellungseinheit an die Steuereinheit und durch Verarbeitung dieser kinematischen Daten in der Steuereinheit einen geeigneten Implantattyp/ eine geeignete Implantatgröße/ eine geeignete Implantatform zu bestimmen.

Es kann ferner zumindest ein Roboterarm vorgesehen sein, welcher eingerichtet bzw. vorbereitet ist, um eine Beweglichkeit des Knies/ Kniegelenks zu untersuchen. Beispielsweise können zwei Roboterarme vorgesehen sein, wobei einer der Roboterarme den Oberschenkel des Patienten greift, einer der Roboterarme den Unterschenkel des Patienten greift, und die beiden Roboterarme eine Flexion bzw. Beugung des Knies bevorzugt über den gesamten Bewegungsumfang durchführen und dabei die kinematischen Daten erfassen. Die Erfassungseinheit kann somit gemäß diesem bevorzugten Ausführungsbeispiel in dem zumindest einen Roboterarm enthalten sein. Alternativ kann auch an einem Patienten angebrachte Sensorik, beispielsweise inertiale Messeinheiten (IMU) zur Erfassung der Kinematik verwendet werden. Wenn eine Apparatur wie ein Roboterarm oder Sensorik wie inertiale Messeinheiten zur Erfassung der kinematischen Daten verwendet wird, können in besonders vorteilhafter Weise standardisierte und vergleichbare kinematische Daten bereitgestellt werden.

Die kinematischen Daten können eine Vielzahl von Datensätzen enthalten, welche aus unterschiedlichen Last- bzw. Belastungssituationen erhalten werden. Beispielsweise kann das Gangbild des Patienten sowohl unter der Last lediglich seines Körpergewichts als auch unter zusätzlichen Lasten, welche der Patient trägt, analysiert werden. Es können beispielsweise Datensätze für das Körpergewicht, eine zusätzliche Last von 5 kg, eine zusätzliche Last von 10 kg, etc. vorliegen. Die kinematischen Daten können somit auch Informationen darüber liefern, wie sich ein betrachteter kinematischer Parameter unter Last mit dem Beuge- bzw. Flexionswinkel des Knies ändert.

Die kinematischen Daten können zumindest einen kinematischen Parameter enthalten oder aus den kinematischen Daten kann zumindest ein kinematischer Parameter bestimmbar sein. Es kann demnach vorgesehen sein, dass die Datenbereitstellungseinheit erfasst/ bereitstellt und/oder die Steuereinheit bestimmt, wie sich der zumindest eine kinematische Parameter bei der Flexion bzw. Beugung des Knies verhält bzw. ändert. Die kinematischen Daten können auch eine Vielzahl von kinematischen Parametern enthalten oder aus den kinematischen Daten kann auch eine Vielzahl von kinematischen Parametern bestimmbar sein. Es kann demnach vorgesehen sein, dass die Datenbereitstellungseinheit erfasst/ bereitstellt und/oder die Steuereinheit bestimmt, wie sich mehrere kinematische Parameter bei der Flexion bzw. Beugung des Knies verhalten bzw. ändern.

Es kann vorgesehen sein, dass die Steuereinheit aus dem zumindest einen kinematischen Parameter unmittelbar bzw. direkt den Implantattyp und die Implantatgröße und die Implantatform bestimmt. Alternativ kann vorgesehen sein, dass die Steuereinheit den zumindest einen kinematischen Parameter verarbeitet, um dem Patienten einen vorbestimmten bzw. vordefinierten kinematischen Phänotypus zuzuordnen. Der Implantattyp/ die Implantatgröße/ die Implantatform kann dann auf der Grundlage des dem Patienten zugeordneten kinematischen Phänotypus bestimmt werden. Der Implantattyp/ die Implantatgröße/ die Implantatform kann somit alternativ auch indirekt aus dem zumindest einen kinematischen Parameter bestimmt werden, indem zuerst der kinematische Phänotypus des Patienten bestimmt wird und dann die Auswahl/ Planung der Knieendoprothese auf der Grundlage des kinematischen Phänotypus erfolgt. Das heißt, der Implantattyp/ die Implantatgröße/ die Implantatform können alternativ auch nicht direkt auf der Grundlage des kinematischen Parameters bestimmt werden. Es gilt jedoch, dass sowohl die direkte als auch die indirekte Bestimmung von Implantattyp/ Implantatgröße/ Implantatform das Ziel haben, durch die prä-operative Analyse und Planung post-operativ für den Patienten eine optimale Kinematik zu erreichen.

Der zumindest eine kinematische Parameter, welcher in Abhängigkeit von dem Flexions- bzw. Beugewinkel des Knies betrachtet wird, kann beispielsweise die Rotation von Tibia gegenüber Femur sein, welche auch als interne-externe Tibiarotation bezeichnet werden kann. Beispielsweise können die Ausprägung der Tibiarotation und/oder die Lokation des Drehpunkts der Tibiarotation betrachtet werden. Alternativ oder zusätzlich kann der zumindest eine kinematische Parameter eine Femurrotation, also eine Rotation des Femurs gegenüber dem Pelvis/ Becken sein. Alternativ oder zusätzlich kann der zumindest eine kinematische Parameter der sogenannte femorale Roll-Back sein, insbesondere dessen Ausprägung und medio-laterale Symmetrie. Alternativ oder zusätzlich kann der zumindest eine kinematische Parameter ein antero-posteriorer Femurshift sein. Darüber hinaus ist es auch denkbar, dass der zumindest eine kinematische Parameter ein Varus bzw. eine Varusfehlstellung (O-Beine) oder ein Valgus bzw. eine Valgusfehlstellung (X-Beine) ist. Beispielsweise kann eine Änderung eines Varus in einen Valgus (oder andersherum) über den Flexions- bzw. Beugewinkel betrachtet/ ausgewertet werden. Auch kann der zumindest eine kinematische Parameter eine Abduktion oder Adduktion im Kniegelenk sein.

Bevorzugt wird zumindest einer der vorgenannten kinematischen Parameter bei der Bestimmung von Implantattyp/ Implantatgröße/ Implantatform berücksichtigt. Es können jedoch auch mehrere der genannten kinematischen Parameter berücksichtigt werden. Von besonderem Vorteil ist es wenn der zumindest eine kinematische Parameter über die komplette Flexions- bzw. Beugebewegung des Knies betrachtet, aufgezeichnet und somit festgehalten wird. Es ist jedoch auch denkbar, dass der zumindest eine kinematische Parameter lediglich bei einer normalen Schrittbewegung, beispielsweise bei einem Doppelschritt, betrachtet wird.

Auch die Bildung von vorbestimmten bzw. vordefinierten kinematischen Phänotypen, auf deren Grundlage die Auswahl bzw. Planung des Implantats erfolgen kann, kann basierend auf dem Verlauf von zumindest einem der vorgenannten kinematischen Parameter über die Extension-Flexion des Kniegelenks erfolgen.

Gemäß einem bevorzugten Ausführungsbeispiel ist die Datenbereitstellungseinheit eingerichtet, Daten über eine Instabilität des Kniegelenks der Steuereinheit bereitzustellen, und die Steuereinheit ist eingerichtet, auch die von der Datenbereitstellungseinheit bereitgestellten Daten über die Instabilität des Kniegelenks zu verarbeiten und den Implantattyp und die Implantatgröße und die Implantatform auch unter Berücksichtigung der Daten über die Instabilität des Kniegelenks zu bestimmen. Die Instabilität des Kniegelenks kann beispielsweise über den sogenannten Kinematic Envelope beschrieben werden. Beim Kinematic Envelope wird zumindest einer (bevorzugt alle) der voranstehend genannten kinematischen Parameter über den gesamten Bewegungsumfang des Kniegelenks, das heißt bei einer Beugung des Kniegelenks von größtmöglicher Extension in größtmögliche Flexion und zurück, erfasst bzw. festgehalten, wobei durch seitliche Krafteinleitung eine Varusbelastung oder eine Valgusbelastung appliziert wird, so dass die medio-laterale Stabilität des Kniegelenks über den gesamten Bewegungsumfang erfasst werden kann. Alternativ kann die Instabilität des Kniegelenks über den sogenannten Lachmann-Test beschrieben werden. Dieser eignet sich insbesondere zur Ermittlung der antero-posterioren Stabilität des Kniegelenks.

Die Steuereinheit kann bei der Verarbeitung der von der Datenbereitstellungseinheit bereitgestellten Daten beispielsweise Simulationsmodelle verwenden bzw. Simulationen durchführen. Außerdem kann vorgesehen sein, dass die Steuereinheit neuronale Netze aufweist und eine künstliche Intelligenz bei der Verarbeitung der Daten unterstützt. Alternativ oder zusätzlich kann vorgesehen sein, dass die Steuereinheit die ihr bereitgestellten Daten, insbesondere die kinematischen Daten, verarbeitet, um dem Patienten einen vorbestimmten bzw. vordefinierten kinematischen Phänotypus zuzuordnen. Es kann bevorzugt sein, wenn die Daten über die Instabilität des Kniegelenks von einem Roboter, beispielsweise einem Roboterarm, aufgenommen werden, so dass standardisierte und vergleichbare Daten vorliegen. Diese Daten, das heißt die Ergebnisse der Stabilitätsuntersuchung bzw. Weichteilcharakterisierung können als Eingabeparameter für Simulationsmodelle verwendet werden, um eine spezifische Weichteilsituation zu berücksichtigen.

Die Datenbereitstellungseinheit kann eingerichtet sein, zusätzlich zu den kinematischen Daten anatomische Daten bereitzustellen, und die Steuereinheit kann eingerichtet sein, die anatomischen Daten und die kinematischen Daten zu verarbeiten und den Implantattyp und die Implantatgröße und die Implantatform basierend auf den anatomischen Daten und den kinematischen Daten zu bestimmen. Unter anatomischen Daten sind bevorzugt Bilddaten, beispielsweise Röntgenbilder und/oder Computertomographie-Daten und/oder Magnetresonanztomographie-Daten zu verstehen. Es ist demnach gemäß einem bevorzugten Ausführungsbeispiel vorgesehen, dass für die prä-operative Planung bzw. Auswahl einer Knieendoprothese sowohl kinematische Daten bzw. Merkmale als auch anatomische Daten bzw. Merkmale verwendet werden.

Ferner kann die Datenbereitstellungseinheit eingerichtet sein, zusätzlich zu den kinematischen Daten Patientendaten bereitzustellen, und die Steuereinheit kann eingerichtet sein, die Patientendaten und die kinematischen Daten zu verarbeiten und den Implantattyp und die Implantatgröße und die Implantatform basierend auf den Patientendaten und den kinematischen Daten zu bestimmen. Die Patientendaten enthalten bevorzugt zumindest einen Parameter aus den Parametern Gewicht, Geschlecht, Größe, Aktivitätslevel, Erkrankung benachbarter Gelenke. Es ist demnach gemäß einem bevorzugten Ausführungsbeispiel vorgesehen, dass für die prä-operative Planung bzw. Auswahl einer Knieendoprothese sowohl kinematische Daten bzw. Merkmale als auch Patientendaten verwendet werden.

Gemäß einem besonders bevorzugten Ausführungsbeispiel ist die Datenbereitstellungseinheit eingerichtet, zusätzlich zu den kinematischen Daten Patientendaten und anatomische Daten bereitzustellen, und ist die Steuereinheit eingerichtet, die Patientendaten, die anatomischen Daten und die kinematischen Daten zu verarbeiten und den Implantattyp/-art und die Implantatgröße und die Implantatform basierend auf den Patientendaten, den anatomischen Daten und den kinematischen Daten zu bestimmen. Es ist demnach gemäß dem besonders bevorzugten Ausführungsbeispiel vorgesehen, dass für die prä-operative Planung bzw. Auswahl einer Knieendoprothese, insbesondere im Hinblick auf Implantattyp/ -größe/ -form, sowohl kinematische Daten bzw. Merkmale als auch anatomische Daten bzw. Merkmale als auch Patientendaten verwendet werden.

Wenn die Datenbereitstellungseinheit der Steuereinheit anatomische Daten bereitstellt, kann vorgesehen sein, dass die anatomischen Daten zumindest einen anatomischen Parameter enthalten bzw. aus den anatomischen Daten zumindest ein anatomischer Parameter bestimmbar ist. Der zumindest eine anatomische Parameter kann somit der Steuereinheit von der Datenbereitstellungseinheit bereitgestellt werden. Alternativ kann die Steuereinheit auch eingerichtet sein, aus den ihr bereitgestellten anatomischen Daten zumindest einen anatomischen Parameter zu bestimmen. Die Steuereinheit kann grundsätzlich auch eingerichtet sein, auf der Grundlage des zumindest einen anatomischen Parameters einen anatomischen Phänotyp des Patienten zu bestimmen. Es kann somit grundsätzlich vorgesehen sein, dass die Bestimmung in der Steuereinheit sowohl den anatomischen Phänotyp als auch den kinematischen Phänotyp des Patienten berücksichtigt. Ein anatomischer Parameter bzw. ein anatomisches Merkmal beschreibt grundsätzlich die statische Gestalt, Lage und Struktur des Kniegelenks, also insbesondere auch von Femur, Tibia und Patella, welche das Kniegelenk zusammen zu einem Gelenk verbinden.

Die Vorrichtung bzw. das System kann grundsätzlich auch eine Fertigungseinheit enthalten, welche eingerichtet ist, das Implantat/ die Knieendoprothese bzw. zumindest einzelne Teile/ Komponenten des Implantats/ der Knieendoprothese auf der Grundlage des von der Steuereinheit bestimmten Implantattyps und der von der Steuereinheit bestimmten Implantatgröße und der von der Steuereinheit bestimmen Implantatform zu fertigen bzw. herzustellen. In diesem Zusammenhang kann es beispielsweise vorgesehen sein, dass ein 3D-CAD-Modell automatisch angepasst wird. Beispielsweise kann die Steuereinheit mit der Fertigungseinheit gekoppelt sein. Die Fertigungseinheit kann etwa eine additive Fertigungseinheit, beispielsweise ein 3D-Drucker sein. Die Steuereinheit kann der Fertigungseinheit CAD-Daten der Knieendoprothese übermitteln und die Fertigungseinheit in Form des 3D-Druckers kann das Implantat in einfacher Weise schnell fertigen. Dabei ist auch eine Fertigung vor Ort im Krankenhaus denkbar, so dass es zwischen Planung und Auswahl des Implantats/ der Knieendoprothese und Durchführen einer Operation durch einen Arzt in vorteilhafter Weise kaum zu einer fertigungsbedingten Verzögerung kommt.

Die vorliegende Offenbarung betrifft weiterhin ein Verfahren zur prä-operativen Planung oder Auswahl eines Implantattyps und einer Implantatgröße und einer Implantatform einer Knieendoprothese, mit den Schritten: Bereitstellen von zumindest patientenspezifischen bzw. patientenindividuellen kinematischen Daten, welche bei einer Flexion bzw. Beugung eines Knies eines Patienten erhalten werden; Verarbeiten der kinematischen Daten und Bestimmen des Implantattyps und der Implantatgröße und der Implantatform basierend auf zumindest den kinematischen Daten; und Bestimmen, dass eine patientenspezifische prä-operative Kinematik des Kniegelenks am besten mit einem sogenannten Medial Pivot Implantat nachgebildet werden kann; oder Bestimmen, dass, falls die patientenspezifische prä-operative Kinematik des Patienten einen ausgeprägten femoralen Roll-Back zeigt, die Kinematik des Kniegelenks am besten mit einem sogenannten PS-Implantat nachgebildet werden kann, welches einen derartigen femoralen Roll-Back erzwingt, unterstützt und fördert.

Das Verfahren weist bevorzugt den Schritt auf: Bestimmen des Implantattyps und der Implantatgröße und der Implantatform derart, dass post-operativ durch eine implantierte Knieendoprothese ein prä-operativer kinematischer Bewegungsablauf bzw. eine prä-operative Kinematik nachgebildet wird.

Bevorzugt enthalten die kinematischen Daten zumindest einen kinematischen Parameter oder ist aus den kinematischen Daten zumindest ein kinematischer Parameter bestimmbar, und das Verfahren weist den Schritt auf: Bestimmen, wie sich der zumindest eine kinematische Parameter bei der Flexion bzw. Beugung des Knies verhält bzw. ändert.

Das Verfahren kann den Schritt aufweisen: Direktes Bestimmen des Implantattyps und der Implantatgröße und der Implantatform aus dem zumindest einen kinematischenParameter. Alternativ kann das Verfahren die Schritte aufweisen: Verarbeiten des zumindest einen kinematischen Parameter; Zuordnen eines vorbestimmten kinematischen Phänotypus dem Patienten; und Indirektes Bestimmen des Implantattyps und der Implantatgröße und der Implantatform auf der Grundlage des dem Patienten zugeordneten kinematischen Phänotypus.

Das Verfahren kann den folgenden Verfahrensschritt umfassen: Aufzeichnen oder Erfassen zumindest eines kinematischen Parameters der folgenden Parameter in Abhängigkeit von dem Flexions- bzw. Beugewinkel des Knies: Rotation von Tibia gegenüber Femur; Interne-externe Femurrotation; Interne-externe Tibiarotation; Ausprägung der Femurrotation; Lokation des Drehpunkts der Femurrotation; Femoraler Roll-Back; Ausprägung des femoralen Roll-Back; Medio-laterale Symmetrie des femoralen Roll-Back; Antero-posteriorer Femurshift; Varus oder Valgus; Abduktion oder Adduktion.

Ferner kann das Verfahren den Schritt enthalten: Bereitstellen von Daten über eine Instabilität des Kniegelenks; Verarbeiten der bereitgestellten Daten über die Instabilität des Kniegelenks; und Bestimmen des Implantattyps und der Implantatgröße und der Implantatform auch unter Berücksichtigung der Daten über die Instabilität des Kniegelenks.

Bevorzugt enthält des Verfahren die Schritte: Bereitstellen von anatomischen Daten zusätzlich zu den kinematischen Daten; Verarbeiten der anatomischen Daten und der kinematischen Daten; und Bestimmen des Implantattyps und der Implantatgröße und der Implantatform basierend auf den anatomischen Daten und den kinematischen Daten. Alternativ kann das Verfahren die Schritte aufweisen: Bereitstellen von Patientendaten zusätzlich zu den kinematischen Daten; Verarbeiten der Patientendaten und der kinematischen Daten; und Bestimmen des Implantattyps und der Implantatgröße und der Implantatform basierend auf den Patientendaten und den kinematischen Daten. Alternativ kann das Verfahren die Schritte aufweisen: Bereitstellen von anatomischen Daten und Patientendaten zusätzlich zu den kinematischen Daten; Verarbeiten der anatomischen Daten, der Patientendaten und der kinematischen Daten; und Bestimmen des Implantattyps und der Implantatgröße und der Implantatform basierend auf den anatomischen Daten, den Patientendaten und den kinematischen Daten.

Weitere bevorzugte Verfahrensschritte ergeben sich für den Fachmann offensichtlich aus der voranstehenden Beschreibung der offenbarungsgemäßen Vorrichtung. Es ist zu berücksichtigen, dass das offenbarungsgemäße Verfahren ein prä-operatives Verfahren ist und keinen Verfahrensschritt einer chirurgischen oder therapeutischen Behandlung des menschlichen Körpers enthält.

### Kurzbeschreibung der Figuren

Die Offenbarung wird nachfolgend mit Hilfe von Figuren weiter erläutert. Es zeigen:
- Fig. 1: eine schematische Ansicht eines ersten Ausführungsbeispiels einer offenbarungsgemäßen Vorrichtung zur prä-operativen Planung oder Auswahl einer Knieendoprothese;
- Fig. 2: eine schematische Ansicht eines zweiten Ausführungsbeispiels einer offenbarungsgemäßen Vorrichtung zur prä-operativen Planung oder Auswahl einer Knieendoprothese.
- Fig. 3: vier vorbestimmte kinematische Die vorliegende Offenbarung betrifft ypen, welche einem Patienten zugeordnet werden können; und
- Fig. 4: eine schematische Ansicht eines dritten Ausführungsbeispiels einer offenbarungsgemäßen Vorrichtung zur prä-operativen Planung oder Auswahl einer Knieendoprothese.

### Figurenbeschreibung

Die Figuren sind lediglich schematischer Natur und dienen ausschließlich dem Verständnis der Offenbarung. Gleiche Elemente sind mit denselben Bezugszeichen versehen. Die Merkmale der einzelnen Ausführungsformen können untereinander ausgetauscht werden.

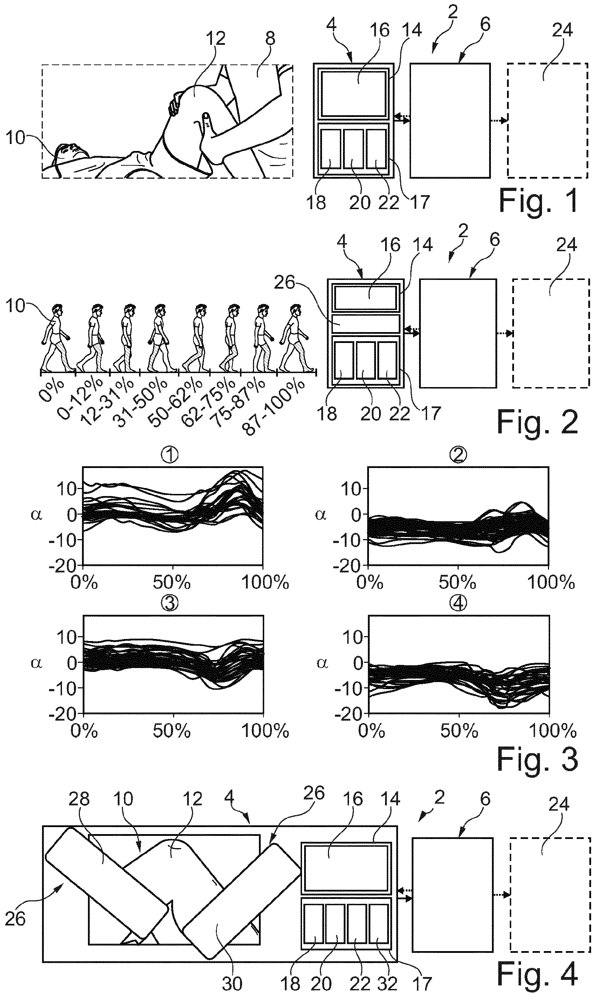

Fig. 1 zeigt eine schematische Ansicht eines ersten Ausführungsbeispiels einer offenbarungsgemäßen Vorrichtung 2 zur prä-operativen Planung oder Auswahl einer Knieendoprothese. Die Vorrichtung 2 weist zumindest eine Datenbereitstellungseinheit 4 und eine Steuereinheit 6 auf. Gemäß dem ersten Ausführungsbeispiel untersucht ein Anwender bzw. Arzt 8 einen Patienten 10, insbesondere eine Beweglichkeit des Kniegelenks 12 des Patienten 10. Dabei führt der Arzt 8 eine Beugung des Kniegelenks 12 von einer größtmöglichen Extension in eine größtmögliche Flexion und zurück durch.

Der Arzt 8 untersucht dabei, wie sich kinematische Parameter bei der Beugung des Kniegelenks 12 verhalten. Unter kinematischen Parametern sind beispielsweise eine interne-externe Tibiarotation, insbesondere im Hinblick auf Ausprägung derselben und/oder Lokation des Drehpunkts, eine Femurrotation, ein femoraler Roll-Back, insbesondere dessen Ausprägung und medio-laterale Symmetrie, ein antero-posteriorer Femurshift, eine Varusfehlstellung oder eine Valgusfehlstellung, eine Abduktion oder eine Adduktion, etc. zu verstehen. Der Arzt 8 stellt gemäß dem ersten Ausführungsbeispiel fest, wie sich zumindest einer der genannten kinematischen Parameter, bevorzugt wie sich mehrere der genannten kinematischen Parameter, bei der Flexion bzw. Beugung des Kniegelenks 12 verhalten bzw. ändern.

Die Datenbereitstellungseinheit 4 weist gemäß dem ersten Ausführungsbeispiel eine Eingabeeinheit 14 in Form einer Benutzerschnittstelle auf. Die Eingabeeinheit 14 hat ein Eingabegerät 16, welches in Fig. 1 beispielsweise in Form eines Touchscreens ausgebildet ist. Der Arzt 8 kann somit seine Beobachtungen in der durchgeführten Untersuchung des Patienten 10 über das Eingabegerät 16 eingeben. Beispielsweise kann die Eingabe derart erfolgen, dass der Arzt 8 geschätzte oder gemessene Winkel oder Verschiebungen zumindest eines der genannten kinematischen Parameter bei verschiedenen Flexions- bzw. Beugungswinkel des Kniegelenks 12 in das Eingabegerät 16 eingibt. Alternativ kann der Arzt 8 zumindest einen, bevorzugt mehrere der genannten kinematischen Parameter bei verschiedenen Flexions- bzw. Beugungswinkel des Kniegelenks 12 bewerten, beispielsweise als gering, normal oder groß. Es kann vorgesehen sein, dass dem Arzt 8 über das als Touchscreen ausgebildete Eingabegerät 16 Fragen zum kinematischen Bewegungsablauf des Kniegelenks 12 des Patienten 10 angezeigt werden, welche der Arzt 8 über vorgegebene Antwortmöglichkeiten entsprechend seinem subjektiven Empfinden in der durchgeführten Untersuchung des Patienten 10 beantworten kann. Schließlich werden die Eingaben bzw. Antworten des Arztes 8 in der Datenbereitstellungseinheit 4, insbesondere in einem Speicher 17 der Datenbereitstellungseinheit 4 als kinematische Daten 18 gespeichert.

In dem Speicher 17 der Datenbereitstellungseinheit 4 sind bevorzugt aber nicht notwendigerweise neben den kinematischen Daten 18 auch noch anatomische Daten 20 und Patientendaten 22 gespeichert. Die anatomischen Daten 20 sind beispielsweise Röntgenbilder, CT-Daten oder MRT-Daten. Die Patientendaten 22 betreffen beispielsweise Gewicht, Geschlecht, Größe, Aktivitätslevel, Erkrankung benachbarter Gelenke, etc. Der Arzt 8 kann auch die Patientendaten 22 über das vorliegend als Touchscreen ausgebildete Eingabegerät 16 eingeben. Auch im Hinblick auf die Patientendaten 22 kann es vorgesehen sein, dass dem Arzt 8 über das als Touchscreen ausgebildete Eingabegerät 16 Fragen zum Patienten 10 angezeigt werden, welche der Arzt 8 über vorgegebene Antwortmöglichkeiten oder über manuelle Eingabe von beispielsweise Gewicht und Größe beantworten kann.

Die Datenbereitstellungseinheit 4 stellt schließlich zumindest die kinematischen Daten 18, bevorzugt die kinematischen Daten 18 zusammen mit den anatomischen Daten 20 und/oder den Patientendaten 22, der Steuereinheit 6 bereit. Die Steuereinheit 6 verarbeitet die ihr bereitgestellten Daten, bevorzugt die kinematischen Daten 18, die anatomischen Daten 20 und die Patientendaten 22. Die Steuereinheit 6 kann sich dabei Simulationsmodellen bedienen, Simulationen oder Berechnungen durchführen, oder auch neuronale Netze aufweisen, so dass auch eine künstliche Intelligenz bei der Verarbeitung der Daten unterstützen kann. Gemäß dem ersten Ausführungsbeispiel bestimmt die Steuereinheit 6 unmittelbar bzw. direkt einen Implantattyp und eine Implantatgröße und eine Implantatform auf der Grundlage der ihr bereitgestellten Daten, insbesondere der kinematischen Daten 18, der anatomischen Daten 20 und der Patientendaten 22. Beispielsweise kann die Steuereinheit 6 eine Implantatgröße der Knieendoprothese auf der Grundlage der ihr bereitgestellten Daten ermitteln. Alternativ oder zusätzlich kann die Steuereinheit 6 die Implantatform der Knieendoprothese bestimmen. Insbesondere kann die Steuereinheit 6 feststellen, dass auf der Grundlage der ihr bereitgestellten Daten abschnittsweise Anpassungen bzw. Änderungen der Implantatform, etwa Verstärkungen, vorzunehmen sind. Alternativ oder zusätzlich kann die Steuereinheit 6 auf der Grundlage der ihr bereitgestellten Daten bestimmen, mit welchem Implantattyp die prä-operative Kinematik des Kniegelenks 12 des Patienten 10 am besten nachgebildet werden kann, beispielsweise mit einem Medial Pivot Implantat oder einem PS-Implantat.

Die Steuereinheit 6 kann das Ergebnis oder die Ergebnisse ihrer Bestimmung zurück an die Datenbereitstellungseinheit 4 senden, welche dieses oder diese über das als Touchscreen ausgebildete Eingabegerät 16 dem Arzt 8 anzeigen kann. Der Arzt 8 kann dann entsprechend für die nachfolgende Operation des Patienten 10 das ihm angezeigte Implantat bzw. die ihm angezeigte Knieendoprothese verwenden, welches bzw. welche optimal an den Patienten 10 angepasst ist, insbesondere an seine Anatomie (Berücksichtigung der anatomischen Daten 20), seine prä-operative Kinematik (Berücksichtigung der kinematischen Daten 18) und an sonstige zu berücksichtigende Eigenschaften oder physischen Merkmale des Patienten (Berücksichtigung der Patientendaten 22). Alternativ kann die Steuereinheit 6 an eine optional vorgesehene Fertigungseinheit 24, welche beispielsweise als 3D-Drucker ausgebildet ist, CAD-Daten der Knieendoprothese oder zumindest eines Bestandteils/ einer Komponente der Knieendoprothese weitergeben und die Fertigungseinheit 24 kann die (Komponente/ den Bestandteil der) Knieendoprothese fertigen bzw. herstellen.

Fig. 2 zeigt eine schematische Ansicht eines zweiten Ausführungsbeispiels einer offenbarungsgemäßen Vorrichtung 2 zur prä-operativen Planung oder Auswahl einer Knieendoprothese. Die Vorrichtung 2 weist auch zumindest eine Datenbereitstellungseinheit 4 und eine Steuereinheit 6 auf. Gemäß dem zweiten Ausführungsbeispiel weist die Datenbereitstellungseinheit 4 eine Erfassungseinheit 26 auf. Die Erfassungseinheit 26 kann als eine oder mehrere Kameras ausgebildet sein, welche das physiologische Gangbild eines Patienten 10 erfassen oder aufzeichnen. Das physiologische Gangbild des Patienten 10 kann in die folgenden Phasen eines Doppelschrittes eingeteilt werden, wobei der Anteil am Doppelschritt in % in Klammern angegeben ist: Anfangskontakt (0%), Belastungsübernahme (0-12%), Mittlere Standphase (12-31%), Standphasenende (31%-50%), Schwungphasenvorbereitung (50-62%), Schwungphasenbeginn (62-75%), mittlere Schwungphase (75-87%) und Schwungphasenende (87-100%). Die Erfassungseinheit 26 erfasst patientenspezifische bzw. patientenindividuelle kinematische Daten 18, insbesondere Fotos oder Videos, über den zeitlichen Ablauf der Gangphasen des Doppelschrittes, und speichert diese kinematischen Daten 18 in dem Speicher 17. Es können auch kinematische Daten 18 bei unterschiedlichen Last- bzw. Belastungssituation (beispielsweise ein Datensatz für das Körpergewicht des Patienten 10, ein Datensatz für eine zusätzliche Last von 5 kg, ein Datensatz für eine zusätzliche Last von 10 kg, etc.) erfasst werden. Auch gemäß der Ausführungsform der Fig. 2 sind in dem Speicher 17 der Datenbereitstellungseinheit 4 bevorzugt aber nicht notwendigerweise neben den kinematischen Daten 18 auch noch anatomische Daten 20 und Patientendaten 22 gespeichert. Der Arzt 8 kann auch hier die Patientendaten 22 über das vorliegend als Touchscreen ausgebildete Eingabegerät 16 der Eingabeeinheit 14 eingeben.

Gemäß der Ausführungsform der Fig. 2 bestimmt die Steuereinheit 6 auf der Grundlage der kinematischen Daten 18 einen Verlauf eines kinematischen Parameters über den zeitlichen Ablauf der Gangphasen des Doppelschrittes. Sämtliche der bereits zur Ausführungsform der Fig. 1 genannten kinematischen Parameter sind in diesem Zusammenhang denkbar. Gemäß der Ausführungsform der Fig. 2 bestimmt die Steuereinheit 6 insbesondere, wie sich Adduktion und Abduktion verhalten, also insbesondere wie sich ein Adduktions- bzw. Abduktionswinkel α über den zeitlichen Ablauf der Gangphasen des Doppelschrittes (von 0% bis 100%) verhält. Wenn die Steuereinheit 6 diese Verarbeitung der kinematischen Daten 18 durchgeführt hat, kann sie dem Patienten 10 einen vorbestimmten bzw. vordefinierten kinematischen Phänotypus zuordnen.

Beispielsweise sind in Fig. 3 vier vorbestimmte bzw. vordefinierte kinematische Phänotypen dargestellt, also vier Beispiele, wie sich der Adduktions- bzw. Abduktionswinkel α über den zeitlichen Ablauf der Gangphasen des Doppelschrittes (von 0% bis 100%) verhalten bzw. ändern kann. Die Steuereinheit 6 bestimmt nun, zu welchem der vier vorbestimmten bzw. vordefinierten kinematischen Phänotypen der Patient am besten passt, indem sie den Adduktions- bzw. Abduktionswinkel α über den zeitlichen Ablauf der Gangphasen des Doppelschrittes des Patienten 10 mit den vier in Fig. 3 gezeigten kinematischen Phänotypen vergleicht, und ordnet dem Patienten 10 einen der vier kinematischen Phänotypen zu. Schließlich wird gemäß der Ausführungsform der Fig. 2 der Implantattyp und die Implantatgröße und die Implantatform auf der Grundlage des dem Patienten 10 zugeordneten kinematischen Phänotypus bestimmt. Beispielsweise kann einem der vier kinematischen Phänotypen ein kleineres Medial Pivot Implantat zugeordnet sein, kann einem der vier kinematischen Phänotypen ein größeres Medial Pivot Implantat zugeordnet sein, kann einem der vier kinematischen Phänotypen ein kleineres PS-Implantat zugeordnet sein, und kann einem der vier kinematischen Phänotypen ein größeres PS-Implantat zugeordnet sein. Gemäß der Ausführungsform der Fig. 2 wird der Implantattyp/ die Implantatgröße/ die Implantatform nicht direkt bestimmt, sondern es wird indirekt aus dem kinematischen Paramter zunächst der kinematische Phänotypus des Patienten 10 bestimmt und die Auswahl/ Planung der Knieendoprothese erfolgt auf der Grundlage des kinematischen Phänotypus.

Auch für die Ausführungsform der Fig. 2 gilt, dass das Ergebnis oder die Ergebnisse der Bestimmung der Steuereinheit 6 dem Arzt 8 angezeigt werden können oder Teile/ Komponenten der Knieendoprothese über die Fertigungseinheit 24 direkt hergestellt werden können.

Fig. 4 zeigt eine schematische Ansicht eines dritten Ausführungsbeispiels einer offenbarungsgemäßen Vorrichtung 2 zur prä-operativen Planung oder Auswahl einer Knieendoprothese. Die Vorrichtung 2 weist auch zumindest eine Datenbereitstellungseinheit 4 und eine Steuereinheit 6 auf. Gemäß dem dritten Ausführungsbeispiel weist die Datenbereitstellungseinheit 4 zwei Roboterarme auf, nämlich einen ersten Roboterarm 28, welcher den Oberschenkel eines Patienten 10 greift, und einen zweiten Roboterarm 30, welcher den Unterschenkel des Patienten 10 greift. Der erste Roboterarm 28 und der zweite Roboterarm 30 sind derart aufeinander abgestimmt, dass sie eine Flexion bzw. Beugung des Kniegelenks 12 des Patienten 10 bevorzugt über den gesamten Bewegungsumfang durchführen können. Die Roboterarme 28, 30 können beispielsweise zusammen mit der Flexion bzw. Beugung des Kniegelenks 12 eine Rotation von Tibia gegenüber Femur durchführen und erfassen/ festhalten, welcher Rotationswinkel in Abhängigkeit von der Flexion bzw. Beugung des Kniegelenks 12 möglich ist. Dabei können die Roboterarme 28, 30 eine Sensorik, beispielsweise einen Kraftsensor, aufweisen, welche eine übermäßige Rotation, die zu einem Reißen von Bändern im Kniegelenk 12 führen könnte, verhindert, insbesondere wenn ein Widerstand zu groß wird. Die beiden Roboterarme 28, 30 sind somit als Erfassungseinheiten 24 anzusehen bzw. weisen Erfassungseinheiten 24 auf. Auch andere der voranstehend genannten kinematischen Parameter können über die beiden Rotoberarme 28, 30 ermittelt bzw. erfasst werden.

Wenn gemäß dem Ausführungsbeispiel der Fig. 4 bei der Beugung des Kniegelenks 12 durch seitliche Krafteinleitung durch die beiden Roboterarme 28, 30 eine Varusbelastung oder ein Valgusbelastung appliziert wird, kann die medio-laterale Stabilität des Kniegelenks 12 über den gesamten Bewegungsumfang erfasst werden. Somit ist es möglich, dass zusätzlich zu den kinematischen Daten 18 noch spezifische Instabilitätsdaten 32 des Kniegelenks 12 ermittelt werden können. Diese Instabilitätsdaten 32 des Kniegelenks 12 können zusammen mit den kinematischen Daten 18, den anatomischen Daten 20 und den Patientendaten 22 in dem Speicher 17 der Datenbereitstellungseinheit 4 gespeichert werden und der Steuereinheit 6 zur weiteren Verarbeitung bereitgestellt werden. Im Übrigen gelten die Ausführungen zu den voranstehenden Ausführungsformen mutatis mutantis für die Ausführungsform der Fig. 4.

### Bezugszeichenliste

- 2: Vorrichtung
- 4: Datenbereitstellungseinheit
- 6: Steuereinheit
- 8: Arzt
- 10: Patient
- 12: Kniegelenk
- 14: Eingabeeinheit
- 16: Eingabegerät
- 17: Speicher
- 18: kinematische Daten
- 20: anatomische Daten
- 22: Patientendaten
- 24: Fertigungseinheit
- 26: Erfassungseinheit
- 28: erster Roboterarm
- 30: zweiter Roboterarm
- 32: Instabilitätsdaten

## Patentansprüche

1. Vorrichtung (2) zur prä-operativen Planung oder Auswahl eines Implantattyps und einer Implantatgröße und einer Implantatform einer Knieendoprothese, mit:
einer Datenbereitstellungseinheit (4), welche eingerichtet ist, zumindest patientenspezifische kinematische Daten (18) bereitzustellen, welche bei einer Beugung eines Kniegelenks (12) eines Patienten (10) erhalten werden; und
einer Steuereinheit (6), welche eingerichtet ist, die von der Datenbereitstellungseinheit (4) bereitgestellten kinematischen Daten (18) zu verarbeiten und den Implantattyp und die Implantatgröße und die Implantatform basierend auf zumindest den kinematischen Daten (18) zu bestimmen, **dadurch gekennzeichnet, dass** die Steuereinheit (6) eingerichtet ist:
zu bestimmen, dass eine patientenspezifische prä-operative Kinematik des Kniegelenks (12) am besten mit einem sogenannten Medial Pivot Implantat nachgebildet werden kann; und
zu bestimmen, dass, falls die patientenspezifische prä-operative Kinematik des Patienten (10) einen ausgeprägten femoralen Roll-Back zeigt, die Kinematik des Kniegelenks (12) am besten mit einem sogenannten PS-Implantat nachgebildet werden kann, welches einen derartigen femoralen Roll-Back erzwingt, unterstützt und fördert.

2. Vorrichtung (2) nach Anspruch 1, wobei die Steuereinheit (6) eingerichtet ist, prä-operativ den Implantattyp und die Implantatgröße und die Implantatform derart zu bestimmen, dass post-operativ durch eine implantierte Knieendoprothese ein prä-operativer kinematischer Bewegungsablauf nachgebildet wird.

3. Vorrichtung (2) nach Anspruch 1 oder 2, wobei die kinematischen Daten (18) zumindest einen kinematischen Parameter enthalten oder aus den kinematischen Daten (18) zumindest ein kinematischer Parameter bestimmbar ist, und die Datenbereitstellungseinheit (4) prä-operativ an die Steuereinheit (6) weitergibt und/oder die Steuereinheit (6) prä-operativ aus den kinematischen Daten (18) bestimmt, wie sich der zumindest eine kinematische Parameter bei der Flexion bzw. Beugung des Kniegelenks (12) ändert.

4. Vorrichtung (2) nach Anspruch 3, wobei
die Steuereinheit (6) eingerichtet ist, prä-operativ aus dem zumindest einen kinematischen Parameter direkt den Implantattyp und die Implantatgröße und die Implantatform zu bestimmen; oder
die Steuereinheit (6) eingerichtet ist, prä-operativ den zumindest einen kinematischen Parameter zu verarbeiten, um dem Patienten (10) einen vorbestimmten kinematischen Phänotypus zuzuordnen und den Implantattyp und die Implantatgröße und die Implantatform auf der Grundlage des dem Patienten (10) zugeordneten kinematischen Phänotypus indirekt zu bestimmen.

5. Vorrichtung (2) nach einem der vorhergehenden Ansprüche 1 bis 4, wobei die Datenbereitstellungseinheit (4) eingerichtet ist, prä-operativ Instabilitätsdaten (32) über eine Instabilität des Kniegelenks (12) der Steuereinheit (6) bereitzustellen, und die Steuereinheit (6) eingerichtet ist, prä-operativ auch die von der Datenbereitstellungseinheit (4) bereitgestellten Instabilitätsdaten (32) über die Instabilität des Kniegelenks zu verarbeiten und den Implantattyp und die Implantatgröße und die Implantatform auch unter Berücksichtigung der Daten über die Instabilität des Kniegelenks (12) zu bestimmen.

6. Vorrichtung (2) nach einem der vorhergehenden Ansprüche 1 bis 5, wobei die Datenbereitstellungseinheit (4) eingerichtet ist, zusätzlich zu den kinematischen Daten (18) anatomische Daten (20) bereitzustellen, und die Steuereinheit (6) eingerichtet ist, die anatomischen Daten (20) und die kinematischen Daten (18) prä-operativ zu verarbeiten und den Implantattyp und die Implantatgröße und die Implantatform basierend auf den anatomischen Daten (20) und den kinematischen Daten (18) prä-operativ zu bestimmen.

7. Vorrichtung (2) nach einem der vorhergehenden Ansprüche 1 bis 5, wobei die Datenbereitstellungseinheit (4) eingerichtet ist, zusätzlich zu den kinematischen Daten (18) Patientendaten (22) bereitzustellen, und die Steuereinheit (6) eingerichtet ist, die Patientendaten (22) und die kinematischen Daten (18) prä-operativ zu verarbeiten und den Implantattyp und die Implantatgröße und die Implantatform basierend auf den Patientendaten (22) und den kinematischen Daten (18) prä-operativ zu bestimmen.

8. Vorrichtung (2) nach einem der vorhergehenden Ansprüche 1 bis 5, wobei die Datenbereitstellungseinheit (4) eingerichtet, zusätzlich zu den kinematischen Daten (18) Patientendaten (22) und anatomische Daten (20) bereitzustellen, und die Steuereinheit (6) eingerichtet ist, die Patientendaten (22), die anatomischen Daten (20) und die kinematischen Daten (18) prä-operativ zu verarbeiten und den Implantattyp und die Implantatgröße und die Implantatform basierend auf den Patientendaten (22), den anatomischen Daten (20) und den kinematischen Daten (18) prä-operativ zu bestimmen.

9. Vorrichtung (2) nach Anspruch 5, wobei die Datenbereitstellungseinheit (4) eingerichtet, zusätzlich zu den kinematischen Daten (18) und den Instabilitätsdaten (32) Patientendaten (22) und anatomische Daten (20) bereitzustellen, und die Steuereinheit (6) eingerichtet ist, die Patientendaten (22), die anatomischen Daten (20), die Instabilitätsdaten (32) und die kinematischen Daten (18) prä-operativ zu verarbeiten und den Implantattyp und die Implantatgröße und die Implantatform basierend auf den Patientendaten (22), den anatomischen Daten (20), den Instabilitätsdaten (32) und den kinematischen Daten (18) prä-operativ zu bestimmen.

10. Verfahren zur prä-operativen Planung oder Auswahl eines Implantattyps und einer Implantatgröße und einer Implantatform einer Knieendoprothese, mit den Schritten:
Bereitstellen von zumindest patientenspezifischen kinematischen Daten (18), welche bei einer Beugung eines Kniegelenks (12) eines Patienten (10) erhalten werden;
Verarbeiten der kinematischen Daten (18) und Bestimmen des Implantattyps und der Implantatgröße und der Implantatform basierend auf zumindest den kinematischen Daten (18); und
Bestimmen, ob eine patientenspezifische prä-operative Kinematik des Patienten (10) einen ausgeprägten femoralen Roll-Back zeigt; und
Bestimmen, dass die patientenspezifische prä-operative Kinematik des Kniegelenks (12) am besten mit einem sogenannten Medial Pivot Implantat nachgebildet werden kann; oder Bestimmen, dass die patientenspezifische prä-operative Kinematik des Kniegelenks (12) am besten mit einem sogenannten PS-Implantat nachgebildet werden kann, welches einen derartigen femoralen Roll-Back erzwingt, unterstützt und fördert.

11. Verfahren nach Anspruch 10, ferner mit dem Schritt: prä-operatives Bestimmen des Implantattyps und der Implantatgröße und der Implantatform derart, dass post-operativ durch eine implantierte Knieendoprothese ein prä-operativer kinematischer Bewegungsablauf nachgebildet wird.

12. Verfahren nach Anspruch 10 oder 11, wobei die kinematischen Daten (18) zumindest einen kinematischen Parameter enthalten oder aus den kinematischen Daten (18) zumindest ein kinematischer Parameter bestimmbar ist, und das Verfahren den Schritt aufweist: prä-operatives Bestimmen, wie sich der zumindest eine kinematische Parameter bei der Flexion bzw. Beugung des Kniegelenks (12) ändert.

13. Verfahren nach Anspruch 12, ferner mit dem Schritt: direktes prä-operatives Bestimmen des Implantattyps und der Implantatgröße und der Implantatform aus dem zumindest einen kinematischen Parameter; oder mit den Schritten: prä-operatives Verarbeiten des zumindest einen kinematischen Parameter; prä-operatives Zuordnen eines vorbestimmten kinematischen Phänotypus dem Patienten (10); und indirektes prä-operatives Bestimmen des Implantattyps und der Implantatgröße und der Implantatform auf der Grundlage des dem Patienten (10) zugeordneten kinematischen Phänotypus.

14. Verfahren nach einem der Ansprüche 10 bis 13, ferner mit dem Schritt: prä-operatives Bereitstellen von Instabilitätsdaten (32) über eine Instabilität des Kniegelenks (12); prä-operatives Verarbeiten der bereitgestellten Instabilitätsdaten (32); und prä-operatives Bestimmen des Implantattyps und der Implantatgröße und der Implantatform auch unter Berücksichtigung der Instabilitätsdaten (32).

## Claims

1. Device (2) for pre-operative planning or selection of an implant type and an implant size and an implant shape of a knee endoprosthesis, comprising:
a data provision unit (4) which is adapted to provide at least patient-specific kinematic data (18) obtained during flexion of the knee joint (12) of a patient (10); and
a control unit (6) that is adapted to process the kinematic data (18) provided by the data provision unit (4) and to determine the implant type and the implant size and the implant shape based on at least the kinematic data (18), **characterized in that** the control unit (6) is configured:
to determine that a patient-specific pre-operative kinematics of the knee joint (12) can be best simulated with a so-called medial pivot implant; and
to determine that, if the patient-specific pre-operative kinematics of the patient (10) shows a distinct femoral roll-back, the kinematics of the knee joint (12) can best be simulated with a so-called PS implant, which enforces, supports and promotes such a femoral roll-back.

2. The device (2) according to claim 1, wherein the control unit (6) is configured to pre-operatively determine the implant type and the implant size and the implant shape such that post-operatively an implanted knee endoprosthesis simulates a pre-operative kinematic motion sequence.

3. The device (2) according to claim 1 or 2, wherein the kinematic data (18) contains at least one kinematic parameter or at least one kinematic parameter can be determined from the kinematic data (18), and the data provision unit (4) pre-operatively passes on to the control unit (6), and/or the control unit (6) pre-operatively determines from the kinematic data (18) how the at least one kinematic parameter changes during flexion or bending of the knee joint (12).

4. The device (2) according to claim 3, wherein
the control unit (6) is adapted to determine pre-operatively the implant type and the implant size and the implant shape directly from the at least one kinematic parameter; or
the control unit (6) is adapted to process the at least one kinematic parameter pre-operatively in order to assign a predetermined kinematic phenotype to the patient (10) and to determine the implant type and the implant size and the implant shape indirectly on the basis of the kinematic phenotype assigned to the patient (10).

5. The device (2) according to any of preceding claims 1 to 4, wherein the data provision unit (4) is adapted to provide pre-operatively instability data (32) about an instability of the knee joint (12) to the control unit (6) and the control unit (6) is adapted to process pre-operatively also the instability data (32) provided by the data provision unit (4) about the instability of the knee joint, and to determine the implant type and the implant size and the implant shape also taking into account the data about the instability of the knee joint (12).

6. The device (2) according to any of preceding claims 1 to 5, wherein the data provision unit (4) is adapted to provide anatomical data (20) in addition to the kinematic data (18) and the control unit (6) is adapted to pre-operatively process the anatomical data (20) and the kinematic data (18) and to pre-operatively determine the implant type and the implant size and the implant shape based on the anatomical data (20) and the kinematic data (18).

7. The device (2) according to any of preceding claims 1 to 5, wherein the data provision unit (4) is adapted to provide patient data (22) in addition to the kinematic data (18), and the control unit (6) is adapted to pre-operatively process the patient data (22) and the kinematic data (18) and to pre-operatively determine the implant type and the implant size and the implant shape based on the patient data (22) and the kinematic data (18).

8. The device (2) according to any of preceding claims 1 to 5, wherein the data provision unit (4) is adapted to provide patient data (22) and anatomical data (20) in addition to the kinematic data (18) and the control unit (6) is adapted to pre-operatively process the patient data (22), the anatomical data (20) and the kinematic data (18) and to pre-operatively determine the implant type and the implant size and the implant shape based on the patient data (22), the anatomical data (20) and the kinematic data (18).

9. The device (2) according to claim 5, wherein the data provision unit (4) is adapted to provide patient data (22) and anatomical data (20) in addition to the kinematic data (18) and the instability data (32) and the control unit (6) is adapted to pre-operatively process the patient data (22), the anatomical data (20), the instability data (32), and the kinematic data (18), and to pre-operatively determine the implant type and the implant size and the implant shape based on the patient data (22), the anatomical data (20), the instability data (32) and the kinematic data (18).

10. A method for the pre-operative planning or selection of an implant type and an implant size and an implant shape of a knee endoprosthesis, comprising the steps of:
Providing at least patient-specific kinematic data (18) obtained during flexion of the knee joint (12) of a patient (10);
Processing the kinematic data (18) and determining the implant type and the implant size and the implant shape based on at least the kinematic data (18); and
Determining if the patient-specific pre-operative kinematics of the patient (10) show a distinct femoral roll-back; and
Determining that the patient-specific pre-operative kinematics of the knee joint (12) can be best simulated with a so-called medial pivot implant; or
Determining that the patient-specific pre-operative kinematics of the knee joint (12) can be best simulated with a so-called PS implant, which forces, supports and promotes such a femoral roll-back.

11. The method according to claim 10, further comprising the step of:
Pre-operatively determining the implant type, the implant size and the implant shape such that a pre-operative kinematic motion sequence is simulated post-operatively by an implanted knee endoprosthesis.

12. The method according to claim 10 or 11, wherein the kinematic data (18) contains at least one kinematic parameter or at least one kinematic parameter can be determined from the kinematic data (18), and the method comprises the step of: pre-operatively determining how the at least one kinematic parameter changes during flexion or bending of the knee joint (12).

13. The method according to claim 12, further comprising the step of: directly determining pre-operatively the implant type and the implant size and the implant shape from the at least one kinematic parameter; or comprising the steps of: pre-operatively processing the at least one kinematic parameter; pre-operatively assigning a predetermined kinematic phenotype to the patient (10); and indirectly pre-operatively determining the implant type and the implant size and the implant shape on the basis of the kinematic phenotype assigned to the patient (10).

14. The method according to any of claims 10_to 13, further comprising the step of: pre-operatively providing instability data (32) about an instability of the knee joint (12); pre-operatively processing the provided instability data (32); and pre-operatively determining the implant type and the implant size and the implant shape, also taking account of the instability data (32).

## Revendications

1. Dispositif (2) de planification ou de sélection pré-opératoire d'un type d'implant et d'une taille d'implant et d'une forme d'implant d'une endoprothèse du genou, avec :
une unité de fourniture de données (4) qui est conçue afin de fournir au moins des données cinématiques (18) spécifiques au patient qui sont obtenues lors d'une flexion d'une articulation du genou (12) d'un patient (10) ; et
un dispositif de commande (6) qui est conçu afin de traiter les données cinématiques (18) fournies par l'unité de fourniture de données (4) et de déterminer le type d'implant et la taille d'implant et la forme d'implant sur la base d'au moins les données cinématiques (18), **caractérisé en ce que** le dispositif de commande (6) est conçu afin de :
déterminer qu'une cinématique pré-opératoire spécifique au patient de l'articulation du genou (12) peut être reproduite au mieux avec un dit implant à pivot médian ; et
déterminer que dans le cas où la cinématique pré-opératoire spécifique au patient du patient (10) montre un rebond fémoral marqué, la cinématique de l'articulation du genou (12) peut être reproduite au mieux avec un dit implant PS qui force, soutient et favorise un tel rebond fémoral.

2. Dispositif (2) selon la revendication 1, dans lequel le dispositif de commande (6) est conçu afin de déterminer de manière pré-opératoire le type d'implant et la taille d'implant et la forme d'implant de telle manière qu'une séquence de mouvement cinématique pré-opératoire soit reproduite de manière post-opératoire par une endoprothèse du genou implantée.

3. Dispositif (2) selon la revendication 1 ou 2, dans lequel les données cinématiques (18) contiennent au moins un paramètre cinématique ou au moins un paramètre cinématique peut être déterminé à partir des données cinématiques (18), et l'unité de fourniture de données (4) transmet de manière pré-opératoire au dispositif de commande (6) et/ou le dispositif de commande (6) détermine de manière pré-opératoire à partir des données (18) cinématiques la manière dont l'au moins un paramètre cinématique se modifie lors du pliage ou de la flexion de l'articulation du genou (12).

4. Dispositif (2) selon la revendication 3, dans lequel
le dispositif de commande (6) est conçu afin de déterminer de manière pré-opératoire à partir de l'au moins un paramètre cinématique directement le type d'implant et la taille d'implant et la forme d'implant ; ou
le dispositif de commande (6) est conçu afin de traiter de manière pré-opératoire l'au moins un paramètre cinématique afin d'associer au patient (10) un phénotype cinématique prédéterminé et de déterminer indirectement le type d'implant et la taille d'implant et la forme d'implant sur la base du phénotype cinématique associé au patient (10).

5. Dispositif (2) selon l'une quelconque des revendications précédentes 1 à 4, dans lequel l'unité de fourniture de données (4) est conçue afin de fournir de manière pré-opératoire des données d'instabilité (32) sur une instabilité de l'articulation du genou (12) au dispositif de commande (6), et le dispositif de commande (6) est conçu afin de traiter de manière pré-opératoire aussi les données d'instabilité (32) fournies par l'unité de fourniture de données (4) sur l'instabilité de l'articulation du genou et de déterminer le type d'implant et la taille d'implant et la forme d'implant aussi en tenant compte des données sur l'instabilité de l'articulation du genou (12).

6. Dispositif (2) selon l'une quelconque des revendications précédentes 1 à 5, dans lequel l'unité de fourniture de données (4) est conçue afin de fournir outre les données cinématiques (18) des données anatomiques (20), et le dispositif de commande (6) est conçu afin de traiter de manière pré-opératoire les données anatomiques (20) et les données cinématiques (18) et de déterminer de manière pré-opératoire le type d'implant et la taille d'implant et la forme d'implant sur la base des données anatomiques (20) et des données cinématiques (18).

7. Dispositif (2) selon l'une quelconque des revendications précédentes 1 à 5, dans lequel l'unité de fourniture de données (4) est conçue afin de fournir outre les données cinématiques (18) des données de patient (22), et le dispositif de commande (6) est conçu afin de traiter de manière pré-opératoire les données de patient (22) et les données cinématiques (18) et de déterminer de manière pré-opératoire le type d'implant et la taille d'implant et la forme d'implant sur la base des données de patient (22) et des données cinématiques (20).

8. Dispositif (2) selon l'une quelconque des revendications précédentes 1 à 5, dans lequel l'unité de fourniture de données (4) est conçue afin de fournir outre les données cinématiques (18) des données de patient (22) et des données anatomiques (20), et le dispositif de commande (6) est conçu afin de traiter de manière pré-opératoire les données de patient (22), les données anatomiques (20) et les données cinématiques (18) et de déterminer de manière pré-opératoire le type d'implant et la taille d'implant et la forme d'implant sur la base des données de patient (22), des données anatomiques (20) et des données cinématiques (18).

9. Dispositif (2) selon la revendication 5, dans lequel l'unité de fourniture de données (4) est conçue afin de fournir outre les données cinématiques (18) et les données d'instabilité (32) des données de patient (22) et des données anatomiques (20), et le dispositif de commande (6) est conçu afin de traiter de manière pré-opératoire les données de patient (22), les données anatomiques (20), les données d'instabilité (32) et les données cinématiques (18) et de déterminer de manière pré-opératoire le type d'implant et la taille d'implant et la forme d'implant sur la base des données de patient (22), des données anatomiques (20), des données d'instabilité (32) et des données cinématiques (18).

10. Procédé de planification ou de sélection pré-opératoire d'un type d'implant et d'une taille d'implant et d'une forme d'implant d'une endoprothèse du genou, avec les étapes suivantes :
la fourniture de données cinématiques (18) au moins spécifiques au patient qui sont obtenues lors d'une flexion d'une articulation du genou (12) d'un patient (10) ;
le traitement des données cinématiques (18) et la détermination du type d'implant et de la taille d'implant et de la forme d'implant sur la base d'au moins les données cinématiques (18) ; et
le fait de déterminer si une cinématique pré-opératoire spécifique au patient du patient (10) montre un rebond fémoral marqué ; et
le fait de déterminer que la cinématique pré-opératoire spécifique au patient de l'articulation du genou (12) peut être reproduite au mieux avec un dit implant à pivot médian ; ou
le fait de déterminer que la cinématique pré-opératoire spécifique au patient de l'articulation du genou (12) peut être reproduite au mieux avec un dit implant PS qui force, soutient et favorise un tel rebond fémoral.

11. Procédé selon la revendication 10, de plus avec l'étape suivante : la détermination pré-opératoire du type d'implant et de la taille d'implant et de la forme d'implant de telle manière qu'une séquence de mouvement cinématique pré-opératoire soit reproduite de manière post-opératoire par une endoprothèse du genou implantée.

12. Procédé selon la revendication 10 ou 11, dans lequel les données cinématiques (18) contiennent au moins un paramètre cinématique ou au moins un paramètre cinématique peut être déterminé à partir des données cinématiques (18), et le procédé présente l'étape suivante : la détermination pré-opératoire de la manière dont l'au moins un paramètre cinématique se modifie lors du pliage ou de la flexion de l'articulation du genou (12).

13. Procédé selon la revendication 12, de plus avec l'étape suivante : la détermination pré-opératoire directe du type d'implant et de la taille d'implant et de la forme d'implant à partir de l'au moins un paramètre cinématique ; ou avec les étapes suivantes : le traitement pré-opératoire de l'au moins un paramètre cinématique ; l'association pré-opératoire d'un phénotype cinématique prédéterminé au patient (10) ; et la détermination pré-opératoire indirecte du type d'implant et de la taille d'implant et de la forme d'implant sur la base du phénotype cinématique associé au patient (10).

14. Procédé selon l'une quelconque des revendications 10 à 13, de plus avec l'étape suivante : la fourniture pré-opératoire de données d'instabilité (32) sur l'instabilité de l'articulation du genou (12) ; le traitement pré-opératoire des données d'instabilité (32) fournies ; et la détermination pré-opératoire du type d'implant et de la taille d'implant et de la forme d'implant aussi en tenant compte des données d'instabilités (32).
